# EUROPEAN PATENT APPLICATION

(11) **EP 4 620 454 A1**
(43) Date of publication of application: **24.09.2025**
(21) Application number: 25163850.8
(22) Date of filing: 14.03.2025
(51) Int. Cl.: A61K 8/81, A61Q 19/08

(54) **USE OF A COMPOSITION CONTAINING POLYVINYL ALCOHOL FOR REDUCING SKIN WRINKLES**

(30) Priority: 20.03.2024 KR 20240038523
(71) Applicant: Amorepacific Corporation, Seoul 04386 (KR)
(72) Inventor: Pi, Bong Soo, Yongin (KR); Kim, Ji Hoon, Yongin (KR); Seo, Jeong Eun, Yongin (KR); Song, Chae Yeon, Yongin (KR); Han, Kyung Sup, Yongin (KR)
(74) Representative: Schön, Christoph

(57) **Abstract**

Use of a composition comprising a polyvinyl alcohol having a number average molecular weight of 30 000 or more, and a water-soluble thickener for reducing skin wrinkles. The effect of reducing skin wrinkles by sustainably contracting the skin can be obtained only by drying after application to the skin without a separate film. The water-soluble thickener may include one or more of natural gums, cellulose, a starch-based thickener, an acrylate-based polymer, a PEG-based polymer, and a PPG-based polymer. In particular, the thickener may be a carbomer.

## Description

### BACKGROUND OF THE INVENTION

### Field of the Invention

The present specification relates to a composition for external use on the skin for reducing skin wrinkles containing polyvinyl alcohol, and discloses a composition for external use on skin, which can contain high molecular weight polyvinyl alcohol to reduce wrinkles by contracting the skin.

### Description of the Related Art

Skin wrinkles are the most noticeable visual feature of skin aging, caused by intrinsic factors such as an increase in age and stress, or by external environmental factors such as air pollution or ultraviolet radiation. Wrinkles occur on each part of the body such as the face, such as the forehead, an area around the eyes, an area between the eyebrows and an area around the mouth, or such as the neck, neck area, elbows, armpits, hands and feet. In most cases, wrinkles usually start appearing around age 30 and increase in number, depth and area with aging. The main cause of wrinkle formation may be believed to be that reactive oxygen species generated in skin tissues during the skin aging process ultimately form crosslinks in collagen fibers and elastic fibers, which occupy most of the skin dermal components, and change the formation and decomposition of these fibers.

Antioxidants such as vitamin E, beta-carotene, vitamin C, and glutathione used to inhibit skin aging, or substances that enhance dermal collagen synthesis, such as radical scavengers and vitamin A, have been used as substances to ameliorate skin wrinkles to date. However, most of these cosmetic active ingredients have strong skin irritation or are themselves unstable in the formulation, and thus disadvantages of causing discoloration, odor, and precipitation, and reducing the inherent activity of the raw materials.

Meanwhile, polyvinyl alcohol is widely known as a film-forming agent, but low molecular weight is usually used, and even when used in mixtures with high molecular weight, it is utilized in pack-type cosmetics that form a film, or is used only for film formation to form a moisturizing film, making it difficult to impart a wrinkle amelioration effect sustainably. Further, existing film-forming agents are primarily intended to reduce the thickness of the film, and thus are insufficient for ameliorating wrinkles by contracting the skin.

### SUMMARY OF THE INVENTION

One aspect of the present disclosure has been made in an effort to provide a composition that is capable of reducing wrinkles by sustainably contracting the skin upon application to the skin.

In one aspect, the present disclosure provides a composition for external use on the skin for reducing skin wrinkles containing: a polyvinyl alcohol having a number average molecular weight of 30,000 or more; and a water-soluble thickener.

In one aspect, the composition according to the present disclosure can reduce skin wrinkles by containing high molecular weight polyvinyl alcohol to sustainably contract the skin.

In another aspect, when the composition according to the present disclosure is applied onto the skin, the pulling force on the skin can be within a suitable range for ameliorating wrinkles, thereby reducing skin wrinkles.

### BRIEF DESCRIPTION OF THE DRAWINGS

FIGS. 1 and 2 are photographs illustrating the effect of a composition according to an embodiment of the present disclosure on reduction of skin wrinkles (nasolabial folds).
FIGS. 3 and 4 are photographs illustrating the effect of a composition according to an embodiment of the present disclosure on reduction of skin wrinkles (neck wrinkles).

### DETAILED DESCRIPTION OF THE INVENTION

Hereinafter, the present disclosure will be described in more detail through the following Examples. However, the following Examples are provided only for illustrative purposes to aid in the understanding of the present disclosure.

Exemplary embodiments of the present disclosure provide a composition for external use on the skin for reducing skin wrinkles containing: a polyvinyl alcohol having a number average molecular weight of 30,000 or more; and a water-soluble thickener.

Another exemplary embodiment of the present disclosure provides a method for reducing skin wrinkles, the method including applying to the skin a composition containing: a polyvinyl alcohol having a number average molecular weight of 30,000 or more; and a water-soluble thickener.

Still another exemplary embodiment of the present disclosure provides a use of a composition containing: a polyvinyl alcohol having a number average molecular weight of 30,000 or more and a water-soluble thickener for preparing a composition for reducing skin wrinkles.

Yet another exemplary embodiment of the present disclosure provides a non-therapeutic use of a composition containing a polyvinyl alcohol having a number average molecular weight of 30,000 or more and a water-soluble thickener for reducing skin wrinkles.

A further exemplary embodiment of the present disclosure provides a composition containing a polyvinyl alcohol having a number average molecular weight of 30,000 or more for reducing skin wrinkles and a water-soluble thickener.

According to the present disclosure, in order to form a film that has the effect of sustainably shrinking skin wrinkles, a mixture of a high molecular weight polyvinyl alcohol having a number average molecular weight of 30,000 or more and a water-soluble thickener is included, so that the pulling force on the skin is appropriate for reducing wrinkles.

In an embodiment, the composition for external use on the skin for reducing skin wrinkles may be applied to a subject having reduced skin elasticity.

In an embodiment, the composition for external use on the skin for reducing skin wrinkles may be applied to a subject having wrinkles on the face, such as the forehead, an area around the eyes, an area between the eyebrows and an area around the mouth (nasolabial folds), or on the neck, neck area, elbows, armpits, hands and feet, and the like.

In an embodiment, the composition for external use on the skin is a general term that may include anything that is applied externally to the skin, and may include cosmetic compositions of various formulations, and the like.

In an embodiment, the composition may be applied to the skin and then dried to contract the skin.

In an embodiment, the polyvinyl alcohol may have a number average molecular weight of 200,000 or less.

For example, the polyvinyl alcohol may have a number average molecular weight of 30,000 or more, 40,000 or more, 50,000 or more, 60,000 or more, 70,000 or more, 80,000 or more, 90,000 or more, 100,000 or more, 110,000 or more, 120,000 or more, 130,000 or more, 140,000 or more, 150,000 or more, 160,000 or more, 170,000 or more, 180,000 or more, or 190,000 or more, and 200,000 or less, 190,000 or less, 180,000 or less, 170,000 or less, 160,000 or less, 150,000 or less, 140,000 or less, 130,000 or less, 120,000 or less, 110,000 or less, 100,000 or less, 90,000 or less, 80,000 or less, 70,000 or less, 60,000 or less, 50,000 or less, or 40,000 or less.

In an embodiment, the water-soluble thickener may include one or more selected from the group consisting of natural gums, cellulose, a starch-derived thickener, an acrylate-based polymer, a PEG-based polymer, and a PPG-based polymer.

In an embodiment, the water-soluble thickener may include one or more selected from the group consisting of carrageenan, xanthan gum, cassia gum, gellan gum, ceratonia siliqua gum, agar, algin, guar gum, pectin, glucomannan, cellulose gum, tamarindus indica seed gum, microcrystalline cellulose, carboxymethyl cellulose, hydroxyethyl cellulose, corn starch, rice starch, sodium acrylate starch, hydrogenated starch, a carbomer, polyacrylic acid, ammonium acryloyldimethyltaurate/vp copolymer, hydroxyethyl acrylate/sodium acryloyldimethyl taurate copolymer, Polyacrylate-13, acrylates/C10-30 alkyl acrylate crosspolymer, PPG-17/IPDI/DMPA copolymer, acrylates/beheneth 25 methacrylate copolymer, and PEG 240/HDI copolymer bis decyltetradeceth 20 ether.

In an embodiment, the polyvinyl alcohol may be contained in an amount of 1 to 20 wt% based on the total weight of the composition.

For example, the polyvinyl alcohol may be contained in an amount of 1 wt% or more, 3 wt% or more, 5 wt% or more, 7 wt% or more, 9 wt% or more, 11 wt% or more, 13 wt% or more, 15 wt% or more, 17 wt% or more, or 19 wt% or more, and 20 wt% or less, 18 wt% or less, 16 wt% or less, 14 wt% or less, 12 wt% or less, 10 wt% or less, 8 wt% or less, 6 wt% or less, 4 wt% or less, or 2 wt% or less based on the total weight of the composition.

In an embodiment, the water-soluble thickener may be contained in an amount of 0.1 to 5 wt% based on the total weight of the composition.

For example, the water-soluble thickener may be contained in an amount of 0.1 wt% or more, 0.5 wt% or more, 1 wt% or more, 1.5 wt% or more, 2 wt% or more, 2.5 wt% or more, 3 wt% or more, 3.5 wt% or more, 4 wt% or more, or 4.5 wt% or more, and 5 wt% or less, 4.5 wt% or less, 4 wt% or less, 3.5 wt% or less, 3 wt% or less, 2.5 wt% or less, 2 wt% or less, 1.5 wt% or less, 1 wt% or less, or 0.5 wt% or less based on the total weight of the composition.

In an embodiment, a weight ratio of the polyvinyl alcohol to the water-soluble thickener may be 1 : 0.01 to 0.6.

For example, the weight ratio of the polyvinyl alcohol to the water-soluble thickener may be 1 : 0.01 or more, 1 : 0.05 or more, 1 : 0.1 or more, 1 : 0.15 or more, 1 : 0.2 or more, 1: 0.25 or more, 1 : 0.3 or more, 1 : 0.35 or more, 1 : 0.4 or more, 1 : 0.45 or more, 1 : 0.5 or more, or 1 : 0.55 or more, and 1 : 0.6 or less, 1 : 0.55 or less, 1 : 0.5 or less, 1 : 0.45 or less, 1 : 0.4 or less, 1 : 0.35 or less, 1 : 0.3 or less, 1 : 0.25 or less, 1 : 0.2 or less, 1 : 0.15 or less, 1 : 0.1 or less, or 1 : 0.05 or less.

When the weight ratio is too low, the film contraction force decreases, and when the weight ratio is too high, a gel with high hardness is formed, so that skin application and spreadability may deteriorate.

In an embodiment, the composition may be applied to the skin at a thickness of 0.1 to 2 mm.

For example, the thickness may be 0.1 mm or more, 0.3 mm or more, 0.5 mm or more, 0.7 mm or more, 0.9 mm or more, 1.1 mm or more, 1.3 mm or more, 1.5 mm or more, 1.7 mm or more, or 1.9 mm or more, and 2 mm or less, 1.8 mm or less, 1.6 mm or less, 1.4 mm or less, 1.2 mm or less, 1 mm or less, 0.8 mm or less, 0.6 mm or less, 0.4 mm or less, or 0.2 mm or less.

The contraction force varies depending on the thickness or application amount, and for example, when the thickness is small because the application amount is excessively low, the film-forming ability may deteriorate and the contraction force may decrease, and in contrast, when the thickness is large because the application amount is excessively high, the skin spreadability deteriorates, so that the composition may not be uniformly applied, and thus, wrinkle amelioration ability may deteriorate because uniform contraction force is not implemented over the entire skin to which the composition is applied.

In an embodiment, the thickness of the composition after drying may be 10 to 25% compared to the thickness of the composition immediately after application of the composition to the skin.

For example, the thickness of the composition after drying may be 10% or more, 12% or more, 14% or more, 16% or more, 18% or more, 20% or more, 22% or more, or 24% or more, and 25% or less, 23% or less, 21% or less, 19% or less, 17% or less, 15% or less, 13% or less, or 11% or less compared to the thickness of the composition immediately after application of the composition onto the skin.

In an embodiment, the composition may sustainably contract the skin for one hour or more.

In an embodiment, the composition may be dried to a moisture content of 3% or less within 15 minutes immediately after application to the skin.

In an embodiment, the usage time of the composition may be from 15 minutes to 24 hours.

For example, the usage time of the composition is the sum of the time required for the composition to be dried and the time required for the skin to be contracted, and may be 15 minutes or more, 30 minutes or more, 1 hour or more, 2 hours or more, 3 hours or more, 4 hours or more, 5 hours or more, 6 hours or more, 7 hours or more, 8 hours or more, 9 hours or more, 10 hours or more, 11 hours or more, 12 hours or more, 13 hours or more, 14 hours or more, 15 hours or more, 16 hours or more, 17 hours or more, 18 hours or more, 19 hours or more, 20 hours or more, 21 hours or more, 22 hours or more, or 23 hours or more, and 24 hours or less, 23 hours or less, 22 hours or less, 21 hours or less, 20 hours or less, 19 hours or less, 18 hours or less, 17 hours or less, 16 hours or less, 15 hours or less, 14 hours or less, 13 hours or less, 12 hours or less, 11 hours or less, 10 hours or less, 9 hours or less, 8 hours or less, 7 hours or less, 6 hours or less, 5 hours or less, 4 hours or less, 3 hours or less, 2 hours or less, 1 hour or less, or 30 minutes or less.

In an embodiment, the composition may be a cosmetic composition.

The appearance of the cosmetic composition contains a cosmetically or dermatologically acceptable medium or base. It is any formulation suitable for topical application, and may be provided in the form of, for example, a solution, a gel, a solid, a kneaded anhydrous product, an emulsion obtained by dispersing an oil phase in the form of an aqueous phase, a suspension, a microemulsion, a microcapsule, microgranules or an ionic (liposome) and a non-ionic vesicular dispersing agent, or in the form of a cream, a skin toner, a lotion, a powder, an ointment, a spray, or a conceal stick. These compositions may be prepared by a typical method in the field. Furthermore, the composition according to the present disclosure may also be used in the form of a foam or an aerosol composition that further contains a compressed propellant.

The cosmetic composition according to an embodiment of the present disclosure may be formulated into a cosmetic such as, for example, a softening lotion, an astringent lotion, a nourishing lotion, a nourishing cream, a massage cream, an essence, an eye cream, an eye essence, a cleansing cream, a cleansing foam, a cleansing water, a pack, a powder, a body lotion, a body cream, a body oil, and a body essence.

When the formulation of the cosmetic composition of the present disclosure is a paste, a cream or a gel, an animal fiber, a vegetable fiber, a wax, paraffin, starch, tragacanth, a cellulose derivative, polyethylene glycol, silicone, bentonite, silica, talc, zinc oxide, or the like may be used as a carrier ingredient.

When the formulation of the cosmetic composition of the present disclosure is a powder or a spray, lactose, talc, silica, aluminum hydroxide, calcium silicate, or a polyamide powder may be used as the carrier ingredient, and in particular, when the formulation of the cosmetic composition of the present disclosure is a spray, the formulation may additionally include a propellant such as a chlorofluorohydrocarbon, propane/butane or dimethyl ether.

When the formulation of the cosmetic composition of the present disclosure is a solution or an emulsion, a solvent, a solubilizer or an emulsifier is used as the carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol or fatty acid esters of sorbitan.

When the formulation of the cosmetic composition of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used as a carrier ingredient.

When the formulation of the cosmetic composition of the present disclosure is a surfactant-containing cleanser, an aliphatic alcohol sulfate, an aliphatic alcohol ether sulfate, sulphosuccinic acid monoester, isethionate, an imidazolinium derivative, methyltaurate, sarcosinate, fatty acid amide ether sulfate, alkylamidobetaine, an aliphatic alcohol, fatty acid glyceride, fatty acid diethanolamide, a vegetable oil, a lanolin derivative, an ethoxylated glycerol fatty acid ester, or the like may be used as the carrier ingredient.

The cosmetic composition of the present disclosure may further contain functional additives and ingredients contained in general cosmetic compositions in addition to the active ingredients. The functional additive may contain an ingredient selected from the group consisting of a water-soluble vitamin, an oil-soluble vitamin, a polymeric peptide, a polymeric polysaccharide, a sphingolipid, and a seaweed extract.

The cosmetic composition of the present disclosure may also be blended with ingredients contained in general cosmetic compositions, if necessary, in addition to the functional additives. Examples of other blended ingredients which may be contained include oil and fat ingredients, a moisturizer, an emollient, a surfactant, organic and inorganic pigments, an organic powder, an ultraviolet absorbent, a preservative, a bactericide, an antioxidant, a plant extract, a pH adjuster, an alcohol, a colorant, a fragrance, a circulation accelerator, a cooling agent, an antiperspirant, purified water, and the like.

Hereinafter, the present disclosure will be described in more detail through the following Examples. However, the following Examples are provided only for illustrative purposes to aid in the understanding of the present disclosure.

### Example

### Preparation of composition for reducing skin wrinkles

As shown in the following Tables 1 and 2, compositions containing PVA with various molecular weights, water, polyvinyl alcohol, and a carbomer were prepared, and then the compositions were applied to a PET film and dried, and the bending angle (contraction force) of the film was measured. Compositions Nos. 7 and 10 were marked with an X because they were too viscous to be prepared.

**[Table 1]**

| Formulations | | | | | |
|---|---|---|---|---|---|
| No. | PVA | Content | Water-soluble polymer | Content | Total |
| 1 | PVA205 (molecular weight ~25,000) (low molecule) | 8.50% | Carbomer | 0.50% | 9.00% |
| 2 | PVA217 (molecular weight ~83,000) (middle molecule) | 8.50% | Carbomer | 0.50% | 9.00% |
| 3 | PVA26-99 (molecular weight ~123,000) (high molecule) | 8.50% | Carbomer | 0.50% | 9.00% |
| 4 | Reagent grade | 8.50% | Carbomer | 0.50% | 9.00% |
| 5 | PVA217 (molecular weight ~83,000) (middle molecule) | 8.90% | Carbomer | 0.10% | 9.00% |
| 6 | PVA217 (molecular weight ~83,000) (middle molecule) | 6.00% | Carbomer | 3.00% | 9.00% |
| 7 | PVA217 (molecular weight ~83,000) (middle molecule) | 4.00% | Carbomer | 5.00% | 9.00% |
| 8 | PVA26-99 (molecular weight ~123,000) (high molecule) | 9.00% | Carbomer | 0.00% | 9.00% |
| 9 | PVA26-99 (molecular weight ~123,000) (high molecule) | 6.00% | Carbomer | 3.00% | 9.00% |
| 10 | PVA26-99 (molecular weight ~123,000) (high molecule) | 4.00% | Carbomer | 5.00% | 9.00% |

**[Table 2]**

| Contraction force | | | |
|---|---|---|---|
| No. | PVA | Room temperature | Refrigeration |
| 1 | PVA205 (molecular weight ~25,000) (low molecule) | 15.5°C | 0°C |
| 2 | PVA217 (molecular weight ~83,000) (middle molecule) | 18°C | 5.5°C |
| 3 | PVA26-99 (molecular weight ~123,000) (high molecule) | 22°C | 14°C |
| 4 | Reagent grade | X | X |
| 5 | PVA217 (molecular weight ~83,000) (middle molecule) | 12.5°C | 3.5°C |
| 6 | PVA217 (molecular weight ~83,000) (middle molecule) | 14.5°C | 4.5°C |
| 7 | PVA217 (molecular weight ~83,000) (middle molecule) | X | X |
| 8 | PVA26-99 (molecular weight ~123,000) (high molecule) | 37°C | 9.5°C |
| 9 | PVA26-99 (molecular weight ~123,000) (high molecule) | 17.5°C | 5°C |
| 10 | PVA26-99 (molecular weight ~123,000) (high molecule) | X | X |

### Test Examples

### Test Example 1 - Pulling force

About 2.7 g of the composition prepared in the Example was applied to a transparent PET film with a size of 46.75 cm² (8.5 cm x 5.5 cm). That is, the composition was applied to a transparent PET film at a concentration of 0.06 g/cm² (a thickness of about 0.1 to 2 mm). Thereafter, the film was sufficiently dried and the degree of bending (angle) of the film was measured.

As a result of the observation, it was confirmed that at room temperature, in the case of composition No. 3 containing the high molecule PVA (molecular weight ~ 123,000) (8.5 wt%) of the Example, the degree of bending was about 22 degrees, and in the case of composition No. 1 containing the low molecule PVA205 (molecular weight ~25,000) (8.5 wt%) of the Comparative Example, the degree of bending was about 15.5 degrees. (see Tables 1 and 2 above)

In addition, in the case of composition No. 2 containing the middle molecule PVA217 (molecular weight ~83,000), it was confirmed that the degree of bending was about 18 degrees. (see Tables 1 and 2 above)

### Test Example 2 - Contraction force sustaining effect

After the experiment in Test Example 1, the sample was stored in a refrigerator (4°C) for 24 hours to confirm how long contraction force was maintained even after 24 hours.

As a result of the observation, it was confirmed that in the case of composition No. 3 containing high molecular PVA (molecular weight ~123,000) (8.5 wt%) of the Example, the degree of bending decreased from about 22 degrees to about 14 degrees, and when low molecular PVA was used, the contraction force was little or weak.

In addition, in the case of composition No. 2 containing the medium molecular PVA217 (molecular weight ~83,000), the degree of bending was 5.5 degrees, which was determined to be in a state where it could be attached to the skin.

Therefore, it was confirmed that only when PVA having an appropriate level of molecular weight was used, optimal contraction force (wrinkle amelioration) and ease of preparation were exhibited.

### Test Example 3 - Skin wrinkle amelioration effect

About 2.7 g of composition No. 3 prepared in the Example was applied to an area of 46.75 cm² (8.5 cm x 5.5 cm) on the skin of a man or woman in his or her 40s. That is, the composition was applied onto the skin (on the nasolabial folds around the mouth and on the neck wrinkle) at a concentration of 0.06 g/cm².

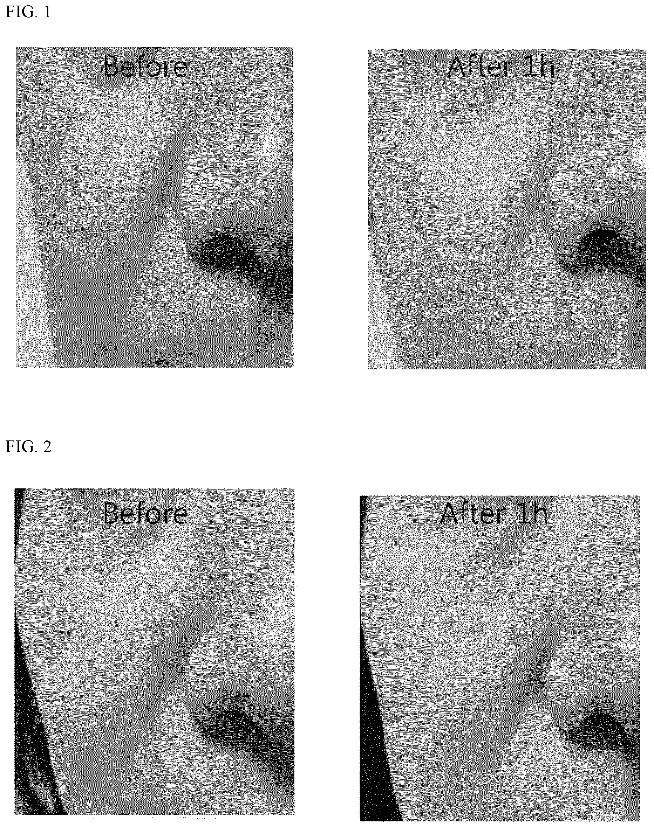

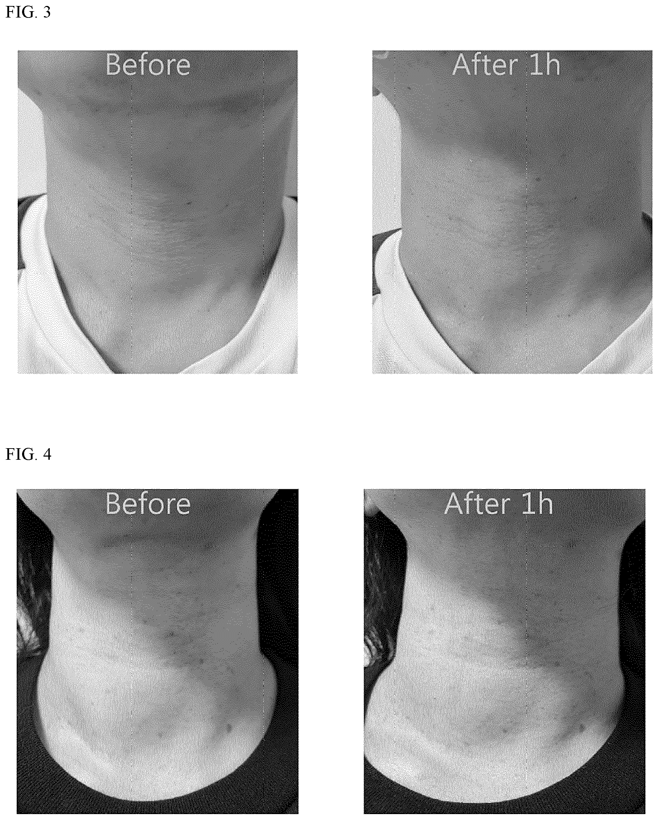

The composition was removed one hour after application, and the effect of ameliorating nasolabial folds and neck wrinkles was determined. Referring to FIGS. 1 and 2 regarding nasolabial folds, it can be confirmed that the deep nasolabial folds have become visibly thinner, and referring to FIGS. 3 and 4 regarding neck wrinkles, it can be confirmed that the number of neck wrinkles has decreased.

### Embodiments

Embodiment 1: A non-therapeutic use of a composition containing a polyvinyl alcohol having a number average molecular weight of 30,000 or more and a water-soluble thickener for reducing skin wrinkles.

Embodiment 2: The non-therapeutic use of Embodiment 1, wherein the composition is applied to the skin and then dried to contract the skin.

Embodiment 3: The non-therapeutic use of Embodiment 1 or 2, wherein the polyvinyl alcohol has a number average molecular weight of 200,000 or less.

Embodiment 4: The non-therapeutic use of any one of Embodiments 1 to 3, wherein the water-soluble thickener includes one or more selected from the group consisting of natural gums, cellulose, a starch-derived thickener, an acrylate-based polymer, a PEG-based polymer, and a PPG-based polymer.

Embodiment 5: The non-therapeutic use of any one of Embodiments 1 to 4, wherein the water-soluble thickener includes one or more selected from the group consisting of carrageenan, xanthan gum, cassia gum, gellan gum, ceratonia siliqua gum, agar, algin, guar gum, pectin, glucomannan, cellulose gum, tamarindus indica seed gum, microcrystalline cellulose, carboxymethyl cellulose, hydroxyethyl cellulose, corn starch, rice starch, sodium acrylate starch, hydrogenated starch, a carbomer, polyacrylic acid, ammonium acryloyldimethyltaurate/vp copolymer, hydroxyethyl acrylate/sodium acryloyldimethyl taurate copolymer, Polyacrylate-13, acrylates/C10-30 alkyl acrylate crosspolymer, PPG-17/IPDI/DMPA copolymer, acrylates/beheneth 25 methacrylate copolymer, and PEG 240/HDI copolymer bis decyltetradeceth 20 ether.

Embodiment 6: The non-therapeutic use of any one of Embodiments 1 to 5, wherein the polyvinyl alcohol is contained in an amount of 1 to 20 wt% based on the total weight of the composition.

Embodiment 7: The non-therapeutic use of any one of Embodiments 1 to 6, wherein the water-soluble thickener is contained in an amount of 0.1 to 5 wt% based on the total weight of the composition.

Embodiment 8: The non-therapeutic use of any one of Embodiments 1 to 7, wherein a weight ratio of the polyvinyl alcohol to the water-soluble thickener is 1: 0.01 to 0.6.

Embodiment 9: The non-therapeutic use of any one of Embodiments 1 to 8, wherein the composition is applied to the skin at a thickness of 0.1 to 2 mm.

Embodiment 10: The non-therapeutic use of any one of Embodiments 1 to 9, wherein a thickness of the composition after drying is 10 to 25% compared to a thickness of the composition immediately after application of the composition to the skin.

Embodiment 11: The non-therapeutic use of any one of Embodiments 1 to 10, wherein the composition sustainably contracts the skin for 1 hour or more.

Embodiment 12: The non-therapeutic use of any one of Embodiments 1 to 11, wherein the composition is dried to a moisture content of 3% or less within 15 minutes.

Embodiment 13: The non-therapeutic use of any one of Embodiments 1 to 12, wherein a usage time of the composition is 15 minutes to 24 hours.

Embodiment 14: The non-therapeutic use of any one of Embodiments 1 to 13, wherein the composition is a cosmetic composition.

## Claims

1. A non-therapeutic use of a composition comprising a polyvinyl alcohol having a number average molecular weight of 30,000 or more and a water-soluble thickener for reducing skin wrinkles.

2. The non-therapeutic use of claim 1, wherein the composition is applied to the skin and then dried to contract the skin.

3. The non-therapeutic use of claim 1 or 2, wherein the polyvinyl alcohol has a number average molecular weight of 200,000 or less.

4. The non-therapeutic use of any one of claims 1 to 3, wherein the water-soluble thickener comprises one or more selected from the group consisting of natural gums, cellulose, a starch-derived thickener, an acrylate-based polymer, a PEG-based polymer, and a PPG-based polymer.

5. The non-therapeutic use of any one of claims 1 to 4, wherein the water-soluble thickener comprises one or more selected from the group consisting of carrageenan, xanthan gum, cassia gum, gellan gum, ceratonia siliqua gum, agar, algin, guar gum, pectin, glucomannan, cellulose gum, tamarindus indica seed gum, microcrystalline cellulose, carboxymethyl cellulose, hydroxyethyl cellulose, corn starch, rice starch, sodium acrylate starch, hydrogenated starch, a carbomer, polyacrylic acid, ammonium acryloyldimethyltaurate/vp copolymer, hydroxyethyl acrylate/sodium acryloyldimethyl taurate copolymer, Polyacrylate-13, acrylates/C10-30 alkyl acrylate crosspolymer, PPG-17/IPDI/DMPA copolymer, acrylates/beheneth 25 methacrylate copolymer, and PEG 240/HDI copolymer bis decyltetradeceth 20 ether.

6. The non-therapeutic use of any one of claims 1 to 5, wherein the polyvinyl alcohol is comprised in an amount of 1 to 20 wt% based on a total weight of the composition.

7. The non-therapeutic use of any one of claims 1 to 6, wherein the water-soluble thickener is comprised in an amount of 0.1 to 5 wt% based on the total weight of the composition.

8. The non-therapeutic use of any one of claims 1 to 7, wherein a weight ratio of the polyvinyl alcohol to the water-soluble thickener is 1 : 0.01 to 0.6.

9. The non-therapeutic use of any one of claims 1 to 8, wherein the composition is applied to the skin at a thickness of 0.1 to 2 mm.

10. The non-therapeutic use of any one of claims 1 to 9, wherein a thickness of the composition after drying is 10 to 25% compared to a thickness of the composition immediately after application of the composition to the skin.

11. The non-therapeutic use of any one of claims 1 to 10, wherein the composition sustainably contracts the skin for 1 hour or more.

12. The non-therapeutic use of any one of claims 1 to 11, wherein the composition is dried to a moisture content of 3% or less within 15 minutes.

13. The non-therapeutic use of any one of claims 1 to 12, wherein a usage time of the composition is 15 minutes to 24 hours.

14. The non-therapeutic use of any one of claims 1 to 13, wherein the composition is a cosmetic composition.
